# EUROPEAN PATENT APPLICATION

(11) **EP 2 818 542 A1**
(43) Date of publication of application: **31.12.2014**
(21) Application number: 12864324.4
(22) Date of filing: 28.12.2012
(51) Int. Cl.: C12M 1/28

(54) **CELL TRAPPING DEVICE**

(30) Priority: 05.01.2012 JP 2012000614
(71) Applicant: Hitachi Chemical Company, Ltd., Chiyoda-ku Tokyo 100-6606 (JP); National University Corporation Tokyo University Of Agriculture and Technology, Tokyo 183-8538 (JP)
(72) Inventor: KIKUHARA Yoshihito, Chikusei-shi Ibaraki 308-0861 (JP); KANBARA Hisashige, Chikusei-shi Ibaraki 308-0861 (JP); SUZUKI Takahiro, Chikusei-shi Ibaraki 308-0861 (JP); MATSUNAGA Tadashi, Fuchu-shi Tokyo 183-8538 (JP); YOSHINO Tomoko, Fuchu-shi Tokyo 183-8538 (JP); HOSOKAWA Masahito, Fuchu-shi Tokyo 183-8538 (JP)
(74) Representative: Hoffmann Eitle
(86) International application number: PCT/JP2012/084210
(87) International publication number: WO 2013/103144

(57) **Abstract**

A cell trapping device includes a housing that includes an inlet opening connected to an inlet line through which a cell dispersion liquid is introduced and an outlet opening connected to an outlet line through which the cell dispersion liquid is discharged; and a filter which is positioned within the housing and includes a trapping region for trapping cancer cells contained in the cell dispersion liquid. The filter is bonded to the housing, at least a part of the trapping region is formed of an observation region for observing the trapping region from the outside, the inlet line and the inlet opening are arranged at outer positions than the observation region when viewed from a normal line direction of the filter, and the inlet line is extended along an in-plane direction of the filter.

## Description

### Technical Field

The present application relates to a cell trapping device that traps cancer cells contained in a cell dispersion liquid.

### Background Art

Cancer ranks high as a cause of death around the world. Particularly, in Japan, the death toll due to cancer reaches 300,000 per year. Thus, there has been a demand for early diagnosis and treatment for that. People have died of cancer mostly due to metastatic relapse of cancer. Metastatic relapse of cancer occurs when cancer cells flow through blood vessels or lymphatic vessels from a primary lesion and adhere to and infiltrate into arterial walls of other organs and form a micrometastatic lesion. Such cancer cells circulating in the body through blood vessels or lymphatic vessels have been referred to as "Circulating Tumor Cells" (hereinafter, sometimes referred to as "CTC").

Blood contains a lot of blood cell components such as red blood cells or white blood cells, and platelets, and the number thereof has been known as 3.5 to 9 x 10⁹ in 1 ml of blood. Only a few CTCs are contained therein. In order to efficiently detect CTCs from the blood cell components, it has been necessary to isolate the blood cell components, and, thus, it has been very difficult to observe and measure the blood cell components.

Many studies have been conducted because detection of CTCs enables early detection of cancer. For example, in Patent Literature 1, it is descried that a cell dispersion liquid containing cancer cells is filtered using a filter made of parylene and having micro through-holes and the cancer cells (CTCs) are efficiently detected by trapping.

### Citation List

### Patent Literature

Patent Literature 1: WO 2010/135603 A

### Summary of Invention

### Technical Problem

Cancer cells such as CTCs have greater sizes than blood cells such as red blood cells, white blood cells, or platelets in blood. Therefore, theoretically, these blood cell components can be removed by applying a mechanical filtering method and cancer cells can be concentrated.

However, in a device equipped with a filter as described in Patent Literature 1, an inlet line and an outlet line through which a cell dispersion liquid is introduced and discharged are protruded from an upper side and a lower side thereof. For this reason, the inlet line or the outlet line becomes an obstacle and an object lens of a microscope cannot be moved to a view position of the filter in some cases. Further, it is difficult to directly fix the device to a stand of the microscope in some cases.

Further, even when the above-described problem is excluded, the device of Patent Literature 1 has a structure in which a resin filter made of parylene is inserted and fixed with polydimethylsiloxane (PDMS) having low stiffness. Thus, when the filter is installed at the device, it is impossible to obtain flatness of the filter. When direct observation is carried out with the microscope, the depth of focus deviates during observation, and, thus, it is necessary to adjust a focus for each object view and working efficiency may decrease.

From the foregoing description, in the device of Patent Literature 1, in order to observe the filter after CTCs are trapped, it is necessary to disassemble the device to take the filter out of the device. For this reason, the taken-out filter may be contaminated with dust or the like in a working environment, which may cause misjudgment. Further, when the device is disassembled, the inside of the device to which a sample derived from the human that can be contaminated with various pathogens or viruses is attached is exposed, and, thus, it is troublesome to ensure safety of work. Furthermore, since the filter is an ultra-thin film, it may be troublesome to handle.

In view of the foregoing, an object of the present application is to provide a cell trapping device that enables observation of cells trapped on a filter without disassembling the device.

### Solution to Problem

According to one embodiment, a cell trapping device including: a housing that includes an inlet opening connected to an inlet line through which a cell dispersion liquid is introduced and an outlet opening connected to an outlet line through which the cell dispersion liquid is discharged; and a filter which is positioned within the housing and includes a trapping region for trapping cancer cells contained in the cell dispersion liquid, in which the filter is bonded to the housing, at least a part of the trapping region is formed of an observation region for observing the trapping region from the outside, the inlet line and the inlet opening are arranged at outer positions than the observation region when viewed from a normal line direction of the filter, and the inlet line is extended along an in-plane direction of the filter.

According to the cell trapping device, it is possible to observe cells trapped on the filter by directly fixing the device to a stand of a microscope without disassembling the device. Further, since the filter is firmly fixed to the housing by bonding, it is possible to filter the cell dispersion liquid at a higher pressure. Thus, it is possible to increase the recovery efficiency of CTCs. Furthermore, since the filter is bonded to the housing of the device, the filter within the device can be flattened. Thus, during microscopic observation, the depth of focus can be uniform, and troublesomeness of adjusting a focus for each object view can be eliminated.

The inlet line and the inlet opening may be arranged at outer positions than the trapping region when viewed from the normal line direction of the filter.

Thus, it is possible to obtain a wider observation region, and it is possible to increase accuracy in detecting CTCs.

Preferably, the filter is substantially flat. Thus, during microscopic observation, the depth of focus can be uniform, and troublesomeness of adjusting a focus for each object view can be eliminated.

Preferably, the filter is formed of a metal. Thus, it is possible to reduce residues of blood cell components and also possible to concentrate CTCs with a high trapping efficiency. Herein, the term "residues" refers to cells which do not pass through the filter but remain on the filter. Since a metal has a high processability, it is possible to increase accuracy in processing the filter. Thus, it is possible to reduce residues of the blood cell components and also possible to obtain a high trapping efficiency of CTCs.

Further, as compared with other materials such as plastic, the metal is rigid, and, thus, even if an external force is applied, the metal can maintain its size or shape. For this reason, it is deemed that blood components (particularly, white blood cells) slightly greater than a hole (through-hole) of the filter are allowed to be deformed and pass through the filter and isolation and concentration can be carried out with a high accuracy. Some of the white blood cells may have sizes equivalent to CTCs, and it is impossible to distinguish the CTCs by only a difference in size with a high accuracy. However, since the white blood cells have a higher deformability than the cancer cells, they can pass through the smaller holes with an external force by way of absorption or pressurization, and, thus, it is possible to isolate the white blood cells from the CTCs.

Furthermore, since the filter is formed of a metal and thus has a high stiffness, it is possible to apply a preset tension when the filter is fixed to the housing. For this reason, the filter can maintain a flat surface without wrinkles or sagging, and it becomes easy to obtain flatness of the filter.

Preferably, at least a part of the housing is substantially transparent in a visible light region. Thus, it is possible to observe the trapping region on the filter from the outside without disassembling the device. Further, since it is not necessary to take the filter out of the device, it is possible to prevent misjudgment caused by contamination of the filter with dust or the like. Furthermore, since it is not necessary to expose the inside of the device to which a sample derived from the human that can be contaminated with various pathogens or viruses is attached, troublesomeness of ensuring safety of work can be reduced. Moreover, troublesome in an operation of handling the ultra-thin film filter can be reduced.

### Advantageous Effects of Invention

According to the present application, it is possible to provide a cell trapping device that enables observation of cells trapped on a filter without disassembling the device.

### Brief Description of Drawings

FIG. 1 is a perspective view illustrating an exemplary embodiment of a cell trapping device.
FIG. 2 is a cross-sectional view taken along a line II-II of FIG. 1.
FIG. 3 is a cross-sectional view illustrating an exemplary embodiment of a cell trapping device.
FIG. 4 is a cross-sectional view illustrating an exemplary embodiment of a cell trapping device.
FIGS. 5(A) and 5(B) are cross-sectional views each illustrating an exemplary embodiment of a cell trapping device.
FIG. 6 is a cross-sectional view illustrating an exemplary embodiment of a cell trapping device.
FIG. 7(A) is a schematic view illustrating an exemplary embodiment of a filter, and FIG. 7(B) is a top view of through-holes of the filter according to the exemplary embodiment.

### Description of Embodiments

Hereinafter, exemplary embodiments of a cell trapping device of the present application will be explained in detail with reference to the accompanying drawings, but the invention is not limited thereto.

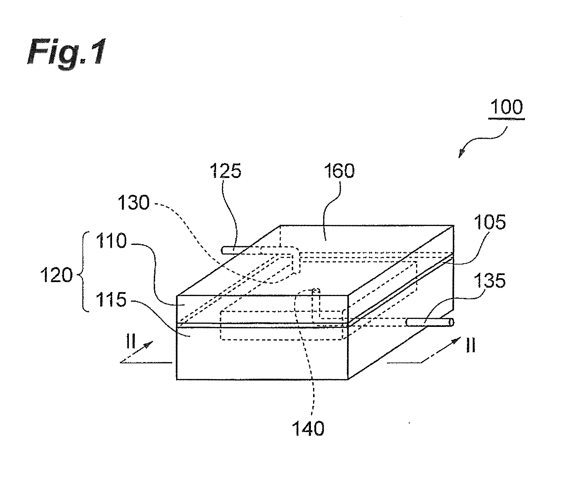

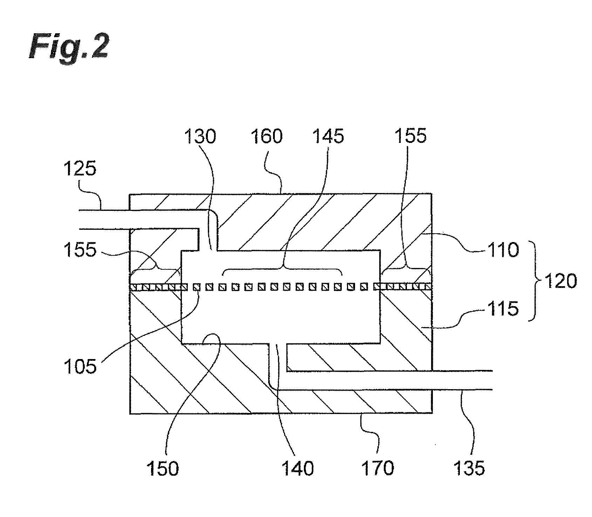

FIG 1 is a perspective view illustrating an exemplary embodiment of a cell trapping device. FIG 2 is a cross-sectional view taken along a line II-II of FIG. 1.

As shown in FIG. 1 and FIG. 2, a cell trapping device 100 includes a housing 120 that includes an inlet opening 130 connected to an inlet line 125 through which a cell dispersion liquid is introduced and an outlet opening 140 connected to an outlet line 135 through which the cell dispersion liquid is discharged; and a filter 105 which is positioned within the housing 120 and includes a trapping region for trapping cancer cells contained in the cell dispersion liquid. At least a part of the trapping region is formed of an observation region 145 for observing the trapping region from the outside, the inlet line 125 and the inlet opening 130 are arranged at outer positions than the observation region 145 when viewed from a normal line direction of the filter 105, and the inlet line 125 is extended along an in-plane direction of the filter 105.

Herein, the in-plane direction refers to all directions in a plane of the filter 105 and all two-dimensional directions on the plane of the filter 105. In the present specification, the expression "along the in-plane direction" refers to a direction having an angle of less than 60°, preferably less than 45°, and more preferably less than 30° with respect to the in-plane direction. The inlet line 125 and the inlet opening 130 are arranged at outer positions than the observation region 145 when viewed from the normal line direction of the filter 105 and the inlet line 125 is extended along the in-plane direction of the filter 105, and, thus, when the observation region 145 on the filter 105 is observed with a microscope through a plane 160 from the outside of the cell trapping device 100, observation can be carried out without disassembling the cell trapping device since there is no structure serving as an obstacle to observation.

Meanwhile, a lower member 115 includes the outlet line 135 and the outlet opening 140. Since the outlet line 135 is positioned at a side surface of the lower member 115, when the observation region 145 is observed with a microscope, the cell trapping device 100 can be directly fixed to a stand of the microscope in a stable manner.

The filter 105 is bonded to an upper member 110 and the lower member 115 in a bonding region 155. Preferably, welding is used as a bonding method. Welding refers to a method of melting materials at a high temperature and directly connecting these materials. The welding may be referred to as fusion, thermo bonding, and heat sealing. The welding may include thermal welding, ultrasonic welding, and high frequency welding. Since the periphery of the filter 105 is welded and fixed through a treatment with heat or ultrasound, the filter 105 can be arranged in a flat state while being applied with a tension without wrinkles or sagging. Thus, the depth of focus is uniform when the filter 105 is observed with a microscope. Therefore, when cells trapped on the filter are observed with a microscope or a magnifying glass, it is not necessary to frequently adjust a focus, and, thus, it is possible to efficiently detect CTCs.

In the present exemplary embodiment, the upper member 110, the filter 105, and the lower member 115 are bonded by welding, respectively. For this reason, it is possible to certainly obtain airtightness of the housing 120, and also possible to obtain a high liquid seal property when the cell dispersion liquid is introduced into the cell trapping device 100. Further, bonding by means of welding can be carried out in a short time, and, thus, productivity is also high.

Further, by selecting materials having high stiffness as the materials of the upper member 110 and the lower member 115, it is possible to suppress deformation of the filter caused by pressurization or heat when the filter 105 is bonded. Herein, a stiffness of the material can be expressed as, for example, Young's modulus. Preferably, Young's modulus of 0.1 GPa or more and more preferably, 1 GPa or more is suitable for materials of the upper member 110 and the lower member 115.

As another bonding method in addition to the welding, a method of coating with an adhesive may be exemplified. However, in the case of bonding with an adhesive, it may be difficult to carry out preferable bonding. For example, when an amount of the adhesive coated is too small, the adhesive may not spread to the whole necessary places. For this reason, complete bonding cannot be carried out, and, thus, a liquid leakage occurs between the upper member 110 and the lower member 115. On the contrary, when an amount of the adhesive coated is too great, the adhesive is coated to be out of a range of necessary positions and may cause clogging in the through-hole of the filter 105. Further, it takes time to coat and cure the adhesive, and, thus, productivity of the cell trapping device 100 may be decreased.

The trapping region of the filter 105 refers to a region where cells can be trapped in a state that the filter 105 is arranged within the housing. The filter 105 may include through-holes in its entire surface or only in its central portion. For example, if the through-holes are present only in a central portion, the region where cells can be trapped is the central portion where the through-holes of the filter 105 are present. Further, if the through-holes are present in the entire surface of the filter 105, since the bonding region 155 between the filter and the housing 120 cannot trap cells, the trapping region is a region of the entire surface of the filter 105 except the bonding region 155. At least a part of the trapping region is the observation region 145, and among cells trapped in the trapping region, cells trapped in the observation region 145 are targets to be observed.

The housing 120 includes the upper member 110 and the lower member 115. A shape of the housing 120 may be a rectangular shape or a cylindrical shape, but is not particularly limited. Preferably, the plane 160 of the upper member 110 and a plane 170 of the lower member 115 are flat and are parallel to each other. In particular, preferably, the plane 160 is smooth. Thus, it becomes easy to directly fix the cell trapping device 100 to a stand of a microscope and observe the observation region 145 on the filter 105 through the plane 160 from the outside of the cell trapping device 100 with the microscope.

A space surrounded by an inner wall 150 of the lower member 115 and the filter 105 is hollow, and within this space, there is no structure such as a support for supporting the filter. For this reason, a flow path for the cell dispersion liquid passing through the filter 105 is not clogged, and, thus, a resistance when the cell dispersion liquid passes through the filter 105 is suppressed to be minimum and the cell dispersion liquid can pass through the filter 105 uniformly. Thus, it is possible to suppress non-uniformity in cell trapping performance or local clogging, so that it is possible to exhibit a stable trapping performance.

The upper member 110 is formed of a substantially transparent material with respect to a light having a wavelength used for detecting CTCs. Examples of a material of the upper member 110 may include glass, quartz glass, an acryl resin, a polymer such as polydimethylsiloxane, but are not limited thereto. In an exemplary embodiment, both of the upper member 110 and the lower member 115 are formed of the above-described material. The upper member 110 and the lower member 115 are not necessarily formed of the same material, but for the sake of easiness in bonding process, preferably, they are formed of the same material. As a material of the upper member 110 and the lower member 115, a low self-fluorescence acryl resin is preferable, and polymethylmethacrylate is particularly preferable for the sake of mass production of the device. Generally, when cancer cells are observed, a staining process is carried out to target cells with a fluorescent reagent and then irradiation with light having a wavelength of 300 to 800 nm in an ultraviolet or visible light region is performed to carry out fluorescent observation. For this reason, as a material of the upper member 110, it is desirable to select a material having a low self-fluorescence emitted by the material itself during irradiation with the light in the above wavelength region (low self-fluorescence). Generally, organic polymers having an aromatic ring, for example, resins such as polystyrene and polycarbonate have a high self-fluorescence and are not suitable for the above purpose in many cases.

A modification example of the cell trapping device of the invention will be explained.

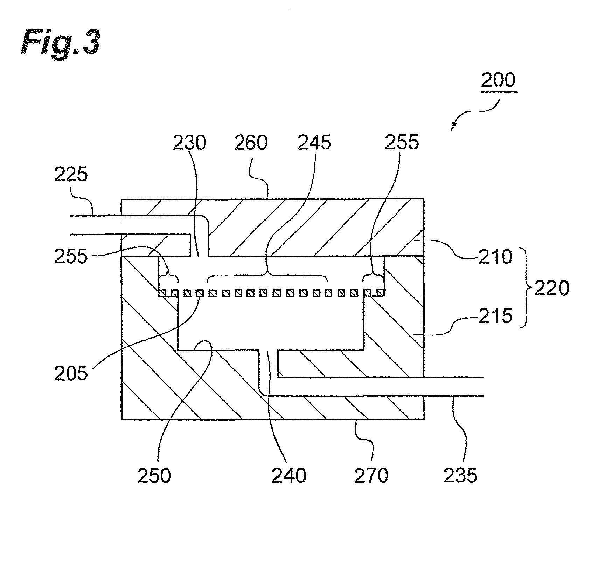

FIG. 3 is a cross-sectional view illustrating an exemplary embodiment of a cell trapping device. In a cell trapping device 200, a stepped portion for holding a filter 205 to a lower member 215 is equipped. The filter 205 is inserted into the stepped portion and bonded to the lower member 215 by welding in a bonding region 255. As for the rest, the cell trapping device 200 is the same as the cell trapping device 100.

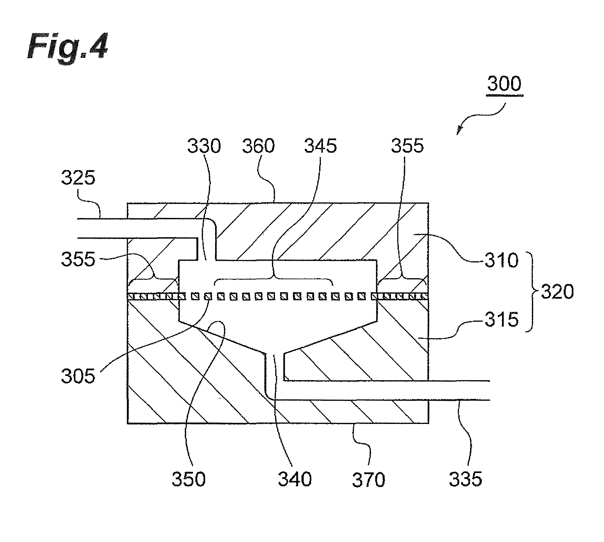

FIG 4 is a cross-sectional view illustrating an exemplary embodiment of a cell trapping device. In a cell trapping device 300, an incline toward an outlet opening 340 is formed at an inner wall 350 of a lower member 315. Thus, collectability of a cell dispersion liquid passing through a filter 305 can be improved and liquid stagnation can be prevented. The incline at the inner wall 350 of the lower member 315 is not limited to a straight line and may be, for example, a circular arc. As for the rest, the cell trapping device 300 is the same as the cell trapping device 100.

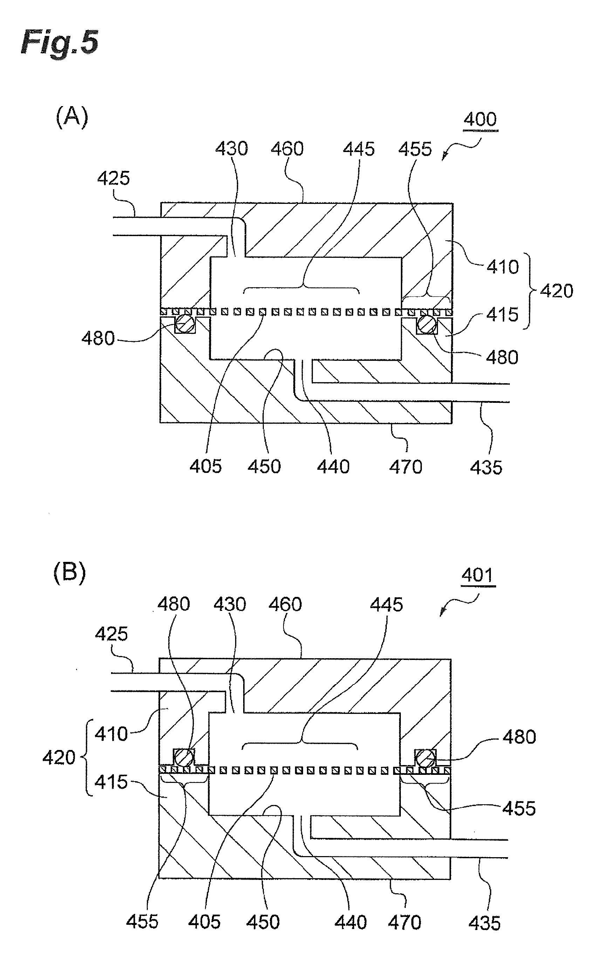

FIG 5(A) is a cross-sectional view illustrating an exemplary embodiment of a cell trapping device. In a cell trapping device 400, a gasket 480 is equipped between a filter 405 and a lower member 415. As for the rest, the cell trapping device 400 is the same as the cell trapping device 100.

FIG. 5(B) is a cross-sectional view illustrating an exemplary embodiment of a cell trapping device. In a cell trapping device 401, the filter 405 is bonded to the lower member 415. Further, the gasket 480 is equipped between the filter 405 and the upper member 410. As for the rest, the cell trapping device 401 is the same as the cell trapping device 100.

The gasket 480 may be used as a fluid resistant seal between an upper member 410, the lower member 415, and the filter 405. Since the gasket 480 is provided, it is possible to more definitely prevent the leakage of the cell dispersion liquid from an outer periphery of the filter 405. Even of an O-ring is used instead of the gasket 480, the same effect can be expected.

Preferably, the gasket 480 is formed of a material having elasticity. Examples of the material of the gasket 480 may include a thermoplastic resin, rubber, elastomer, and the like.

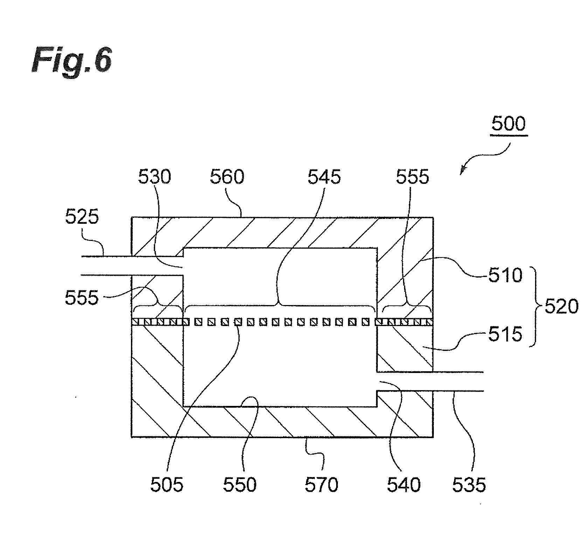

FIG. 6 is a cross-sectional view illustrating an exemplary embodiment of a cell trapping device. In a cell trapping device 500, an inlet line 525 is arranged at a side surface of an upper member 510, that is, at an outer position than a trapping region when viewed from a normal line direction of a filter 505. In this configuration, when an observation region 545 on the filter 505 is observed with a microscope through a plane 560 from the outside of the cell trapping device 500, there is no structure serving as an obstacle to observation. Therefore, it is possible to set the observation region 545 to be wider and also possible to increase an area of the observation region 545 up to be equal to the trapping region of the filter 505 in maximum. Further, an outlet line 535 is arranged at a side surface of a lower member 515. As for the rest, the cell trapping device 500 is the same as the cell trapping device 100.

A filter will be explained hereinafter.

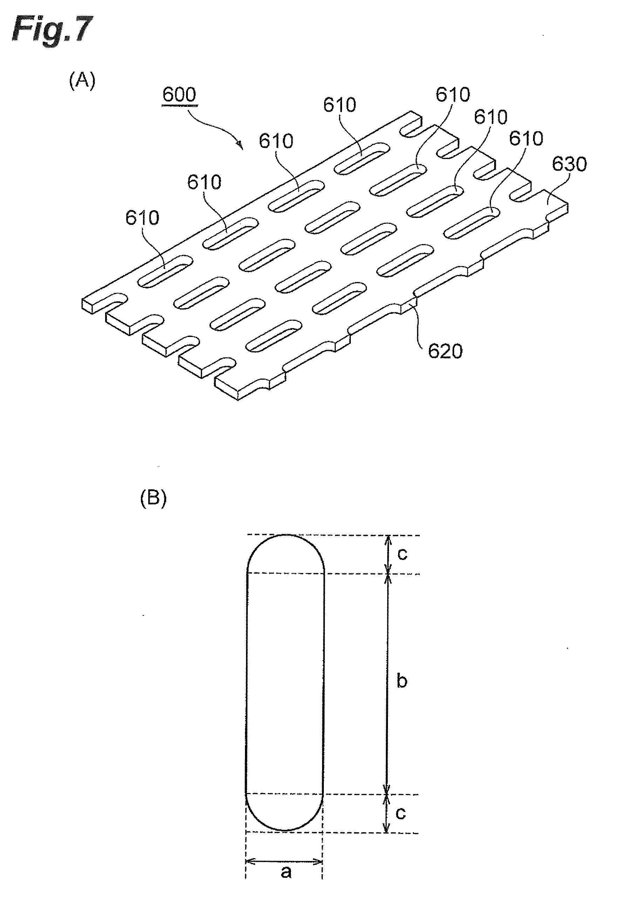

FIG 7(A) is a schematic view illustrating an exemplary embodiment of a filter. A filter 600 is formed of a substrate 620 including multiple through-holes 610, and CTCs are trapped on a surface of a plane 630. The through-holes 610 may be arranged in lines as shown in FIG. 7(A) and may be arranged in lines in a zigzag pattern and may be randomly arranged.

FIG. 7(B) is a top view of the through-holes 610 of the filter 600. An opening of the through-hole 610 has a shape of combined two semicircles each having the radius c and adjacent to a short side of a rectangle having short sides a and long sides b. In an exemplary embodiment, a, b, and c are 8, 37, and 4 µm, respectively.

In an exemplary embodiment, the substrate 620 of the filter is formed of a natural polymer such as cotton and hemp, a synthetic polymer such as nylon, polyester, polyacrylonitrile, polyolefin, halogenated polyolefin, polyurethane, polyamide, polysulfone, polyethersulfone, poly(meth)acrylate, a halogenated polyolefin ethylene-polyvinyl alcohol copolymer, and a butadiene-acrylonitrile copolymer, and mixtures thereof. Further, examples of a material thereof may include a metal, ceramics, and composite materials thereof.

In an exemplary embodiment, a material of the substrate 620 of the filter is a metal. Examples of the metal may include precious metals such as gold and silver, base metals such as copper, aluminum, tungsten, nickel, and chromium, and alloys thereof, but is not limited thereto. A metal may be used alone or in any alloy with other metals or metal oxide for providing functionality. More preferably, a metal containing nickel, stainless steel, or titanium, which is not easily subjected to oxidation or corrosion, as a main component may be used. Herein, the term "main component" refers to a component that accounts for 50% or more of a material forming the substrate. Through-holes may be formed on the metal using a photolithography method or the like.

An opening of a through-hole may have, for example, a circular shape, an oval shape, a rectangular shape, a round rectangular shape, a polygonal shape, and the like. A circular shape, a rectangular shape, or a round rectangular shape is preferable to efficiently trap cancer cells. Further, particularly, a rectangular shape or a round rectangular shape is preferable to prevent clogging in a filter.

Generally, CTCs have a diameter of 10 µm or more. Herein, a diameter of a cell refers to a length of the longest straight line of straight lines connecting any two points on a contour of the cell when observed with a microscope. Therefore, in view of penetrability of blood cells and trapping performance of CTCs, an average hole diameter of through-holes is preferably 5 to 15 µm, more preferably 6 to 12 µm, and particularly preferably 7 to 10 µm. In the present specification, when an opening has a shape of an oval shape, a rectangular shape, and a polygonal shape except a circular shape, a hole diameter is the maximum value of a diameter of a sphere which can pass through each through-hole. For example, when an opening has a rectangular shape, a hole diameter of a through-hole is a length of a short side of the rectangular shape, and when an opening has a polygonal shape, a hole diameter is a diameter of an inscribed circle of the polygonal shape. When an opening has a rectangular shape or a round rectangular shape, even if CTCs or white blood cells are trapped by through-holes, there is formed a gap in a long side direction of the opening. Liquid can pass through this gap, and, thus, clogging in the filter can be prevented.

An average aperture ratio of the through-holes of the filter is preferably 0.1 to 50%, more preferably 0.5 to 40%, particularly preferably 1 to 30%, and most preferably 1 to 10%. Herein, the aperture ratio refers to an area of the thorough-holes with respect to the area of the entire filter. Preferably, in view of prevention of clogging, the average aperture ratio is as high as possible, but if it is higher than 50%, strength of the filter may be decreased or it may be difficult to process. Further, if it is lower than 0.1%, the filter is easily clogged, and, thus, a cancer cell trapping performance of the filter may decrease.

A thickness of the substrate of the filter is preferably 3 to 100 µm, more preferably 5 to 50 µm, and particularly preferably 10 to 30 µm. If the thickness of the substrate is less than 3 µm, strength of the filter may be decreased, and, thus, it may be difficult to handle. On the other hand, if the thickness of the substrate is more than 100 µm, more materials than necessary may be consumed or it may take a long time to process, and, thus, it may be unfavorable in terms of costs or precision processing may be impossible.

A flatness of a surface of the filter may vary depending on a magnification of a microscope used for observation, but preferably, it may be 10 µm or less as R_{z} (maximum height roughness) defined by JIS B0601-2001.

Further, a manufacturing method of a filter according to the present exemplary embodiment will be explained. The manufacturing method of a filter is not specifically limited, and may include a manufacturing method performing etching or electroplating using, for example, a photolithography method. The manufacturing method using the photolithography method will be explained hereinafter. First, a photo-sensitive resist film (photo-sensitive layer) is bonded onto a support formed of stainless steel or the like. Then, a mask having a pattern in the shape of the openings of the through holes of the filter is fixed onto the photo-sensitive layer. Then, light (active light) is irradiated on the mask. After irradiation with the light, if the support is present on the photo-sensitive layer, it is removed, a non-exposed portion is removed and developed by wet development using a developing liquid such as an alkaline aqueous solution, a water-based developing liquid, and an organic solvent, or dry development, and a resist pattern is formed. Thereafter, using the developed resist pattern as a mask, plating is carried out onto a non-masked and exposed substrate. Examples of a plating method may include copper plating, tin-lead plating, nickel plating, gold plating, and the like. After plating, if a plating layer is obtained by peeling the plating layer off from the support and the photosensitive layer. This plating layer is a filter. A surface of the obtained filter may be roughened. A method of a roughening process may include chemical etching using an acidic or alkaline aqueous solution, or a physical process such as sand blast.

A method of using a cell trapping device will be explained.

As a cell dispersion liquid, blood, lymph fluid, tissue fluid, cord blood stagnating in the bone marrow, the spleen, and the liver can be used, but it is most convenient to use peripheral blood circulating in the body. Detecting presence of CTCs in peripheral blood is a useful means for determining a pathologic development of cancer.

When presence or absence of CTCs in a cell dispersion liquid is detected, the cell dispersion liquid is introduced through an inlet line of the cell trapping device, cells containing CTCs are concentrated on a filter, and whether or not CTCs are present in the concentrated cells is checked. When the cell dispersion liquid is introduced through the inlet line, for example, a method using pressurization or depressurization, or a method using a peristaltic pump may be applied. Further, an area of a trapping region of the filter may be 0.25 to 10 cm², for example, if CTCs are concentrated from 1 mL of blood.

If CTCs are concentrated by the above-described method, blood cells such as white blood cells as well as the CTCs are concentrated at the same time. For this reason, it is necessary to check whether or not epidermal cells derived from a primary lesion of cancer are contained in the collected cells. For example, CTCs are concentrated by the above-described method and then stained with an antibody against a fluorescence-labeled epithelial cell marker, and, thus, it is possible to confirm that they are epithelial cells. As the antibody against the epithelial cell marker, an anti-cytokeratine antibody may be used.

For example, the concentrated cells can be stained and observed as follows. After concentration of the cells, an anti-cytokeratine antibody solution is introduced through an inlet line of the cell trapping device and settled for a certain period of time. Then, a cleaning buffer is introduced through the inlet line of the cell trapping device and a non-reacted antibody is cleaned and removed. Then, the cell trapping device is directly fixed to a stand of a microscope, and fluorescent microscopic observation is carried out. Before the antibody solution is introduced into the cell trapping device, a blocking buffer for suppressing a non-specific reaction of the antibody may be introduced.

Further, the cells concentrated by the above-described method are collected and a gene analysis is conducted, and therefore, it is possible to confirm presence of cancer cells. The cells can be collected by, for example, introducing a buffer through an outlet line of the cell trapping device and collecting the buffer through the inlet line. For example, through analysis of modification of genes such as p53, K-RAS, H-RAS, N-RAS, BRAF, and APC, it is possible to confirm presence of cancer cells. Further, an analysis result of these genes may be used later for a therapeutic decision for a patient. Otherwise, it is possible to confirm presence of cancer cells by measuring activity of telomerase of the cells concentrated by the above-described method.

After detection of presence or absence of CTCs in the cell dispersion liquid is ended, a cleaning buffer is introduced through the outlet line of the cell trapping device and discharged through the inlet line, and, thus, cells trapped by the filter can be cleaned and removed and the cell trapping device can be used again.

### Reference Signs List

100,200, 300, 400, 401, 500: Cell trapping device
105, 205, 305, 405, 505, 600: Filter
110,210, 310, 410, 510: Upper member
115, 215, 315, 415, 515: Lower member
120, 220, 320, 420, 520: Housing
125, 225, 325, 425, 525: Inlet line
130, 230, 330, 430, 530: Inlet opening
135,235,335,435, 535: Outlet line
140, 240, 340, 440, 550: Outlet opening
145,245, 345, 445, 545: Observation region
150,250,350,450,550: Inner wall of lower member
155, 255, 355, 455, 555: Bonding region
160, 170, 260, 270, 360, 370, 460, 470, 560, 570, 630: Plane
480: Gasket
610: Through-holes
620: Substrate
a: Short side
b: Long side
c: Radius.

## Claims

1. A cell trapping device comprising:
a housing that includes an inlet opening connected to an inlet line through which a cell dispersion liquid is introduced and an outlet opening connected to an outlet line through which the cell dispersion liquid is discharged; and
a filter which is positioned within the housing and includes a trapping region for trapping cancer cells contained in the cell dispersion liquid,
wherein the filter is bonded to the housing,
at least a part of the trapping region is formed of an observation region for observing the trapping region from the outside,
the inlet line and the inlet opening are arranged at outer positions than the observation region when viewed from a normal line direction of the filter, and
the inlet line is extended along an in-plane direction of the filter.

2. The cell trapping device according to claim 1,
wherein the inlet line and the inlet opening are arranged at outer positions than the trapping region when viewed from the normal line direction of the filter.

3. The cell trapping device according to claim 1 or 2,
wherein the filter is substantially flat.

4. The cell trapping device according to any one of claims 1 to 3,
wherein the filter is formed of a metal,

5. The cell trapping device according to any one of claims 1 to 4,
wherein at least a part of the housing is substantially transparent in a visible light region.

## Amended claims

### Amended claims under Art. 19.1 PCT

**1.** A cell trapping device comprising:
a housing that includes an upper member and an lower member, wherein the upper member includes an inlet opening connected to an inlet line through which a cell dispersion liquid is introduced, and the lower member which includes an outlet opening connected to an outlet line through which the cell dispersion liquid is discharged; and
a filter which is positioned within the housing and includes a trapping region for trapping cancer cells contained in the cell dispersion liquid,
wherein the filter is bonded to the housing,
at least a part of the trapping region is formed of an observation region for observing the trapping region from the outside,
the inlet line and the inlet opening are arranged at outer positions than the observation region when viewed from a normal line direction of the filter, and
the inlet line is extended along an in-plane direction of the filter.

**2.** The cell trapping device according to claim 1,
wherein the inlet line and the inlet opening are arranged at outer positions than the trapping region when viewed from the normal line direction of the filter.

**3.** The cell trapping device according to claim 1 or 2,
wherein the filter is substantially flat.

**4.** The cell trapping device according to any one of claims 1 to 3,
wherein the filter is formed of a metal,

**5.** The cell trapping device according to any one of claims 1 to 4,
wherein at least a part of the housing is substantially transparent in a visible light region.

**6.** The cell trapping device according to any one of claims 1 to 5,
wherein the upper member and the lower member are bonded by welding.

Statement under Art. 19.1 PCT
The feature of Claim 1 has been clarified so that the housing has an "upper member includes an inlet opening connected to an inlet line" and a "lower member includes an outlet opening connected to an outlet line". The invention according to Claim 1 as amended achieves an effect whereby it is possible to filter cell dispersion liquid at high pressure since the filter is firmly secured to the housing by bonding,

The feature of Claim 6 has been clarified so that "the upper member and the lower member are bonded by welding". The invention according to Claim 6 as amended achieves an effect whereby airtightness of the housing can be obtained with certainty, and high liquid sealing performance within the cell trapping device can be ensured without using a sealing member or the like.
